# EUROPÄISCHE PATENTSCHRIFT

(11) **EP 3 668 848 B1**
(45) Veröffentlichungstag und Bekanntmachung des Hinweises auf die Patenterteilung: **30.06.2021**
(21) Anmeldenummer: 18752504.3
(22) Anmeldetag: 16.08.2018
(51) Int. Cl.: C07D 317/36

(54) **VERFAHREN ZUR HERSTELLUNG VON (METH)ACRYLATEN VON GLYCERINCARBONAT**
PROCESS FOR THE PREPARATION (METH)ACRYLATES OF GLYCEROL CARBONATES
PROCÉDÉ POUR LA PREPARATION DES GLYCÉRINE CARBONATES DES (METH)ACRYLATES

(30) Priorität: 17.08.2017 EP 17186588
(43) Veröffentlichungstag der Anmeldung: 24.06.2020
(73) Patentinhaber: BASF SE, 67056 Ludwigshafen am Rhein (DE)
(72) Erfinder: BLANCHOT, Mathieu, 67056 Ludwigshafen (DE); MEISENBURG, Uwe, 67056 Ludwigshafen (DE); MAURER,Steffen, 67056 Ludwigshafen (DE); PETZOLDT, Jochen, 67056 Ludwigshafen (DE); HOEFENER, Tobias, 40589 Düsseldorf-Holthausen (DE); BREITSCHEIDEL, Boris, 67056 Ludwigshafen (DE); MISSKE, Andrea, 67056 Ludwigshafen (DE); FLEISCHHAKER, Friederike, 67056 Ludwigshafen (DE); KALLER, Martin, 67056 Ludwigshafen (DE); FLECKENSTEIN, Christoph, 67056 Ludwigshafen (DE); STENGEL, Ulrik, 67056 Ludwigshafen (DE); NAIR, Ritesh, 67056 Ludwigshafen (DE)
(74) Vertreter: BASF IP Association
(86) Internationale Anmeldenummer: PCT/EP2018/072166
(87) Internationale Veröffentlichungsnummer: WO 2019/034716

(56) Entgegenhaltungen:
- WO-A1-2013/129486
- DE-A1- 10 355 830

## Beschreibung

Die vorliegende Erfindung betrifft ein Verfahren zur Herstellung von Estern der (Meth)acrylsäure durch Umsetzung von (Meth)acrylsäure oder einem Ester davon mit Glycerincarbonat in Gegenwart wenigstens eines die Veresterungsreaktion katalysierenden Enzyms in Abwesenheit eines Lösungsmittels bei einer Reaktionstemperatur von 10 bis 150 °C.

Verfahren zur Herstellung von Estern der (Meth)acrylsäure oder einem Derivat davon mit Glycerincarbonat sind dem Fachmann aus dem Stand der Technik bereits bekannt.

WO 2013/129486 A1 offenbart ein Verfahren zur Herstellung eines Esters der Acrylsäure und Glycerincarbonat durch Umsetzung von Glycerincarbonat mit Acrylsäure-vinyl-ester in Gegenwart einer die Umesterungsreaktion katalysierenden Lipase und tert.-Butanol als Lösungsmittel.

N. Bassam et al., Green Chemistry, 2013, 15, 1900 bis 1909, offenbaren ein Verfahren zur Herstellung von Acrylsäureglycerincarbonatester durch Umsetzung von Glycerincarbonat mit Acrylsäurechlorid in Gegenwart von Triethylamin in Dichlormethan als Lösungsmittel.

DE 103 55 830 A1 betrifft ein Verfahren zur Herstellung von Glycerincarbonatmethacrylat in Gegenwart von Metall-Chelat-Katalysatoren vom Typ Metallion-1,3-Diketonat.

Die bekannten Verfahren zur Herstellung von (Meth)acrylsäureestern von Glycerincarbonat können bezüglich ihrer Raum-Zeit-Ausbeute noch verbessert werden. Des Weiteren ist die Reinheit der durch die Verfahren des Standes der Technik erhaltenen (Meth)acrylsäureester mit Glycerincarbonat noch zu verbessern.

Aufgabe der vorliegenden Erfindung ist es demnach, ein Verfahren zur Herstellung von Estern der (Meth)acrylsäure mit Glycerincarbonat bereitzustellen, welches die oben genannten Nachteile nicht aufweist. Insbesondere soll das Verfahren die gewünschten Produkte in hoher Ausbeute, Selektivität und hoher Reinheit zugänglich machen.

Diese Aufgaben werden gelöst durch das erfindungsgemäße Verfahren zur Herstellung von Estern der (Meth)acrylsäure oder einem Derivat davon durch Umsetzung von (Meth)acrylsäure oder einem Ester davon mit Glycerincarbonat in Gegenwart wenigstens eines die Veresterungsreaktion katalysierenden Enzyms in Abwesenheit eines Lösungsmittels bei einer Reaktionstemperatur von 10 bis 150 °C.

Diese Aufgaben werden speziell gelöst durch das erfindungsgemäße Verfahren zur Herstellung von Estern der (Meth)acrylsäure durch Umsetzung von (Meth)acrylsäure oder C₁₋₁₂-Alkyl(meth)acrylat mit Glycerincarbonat in Gegenwart wenigstens eines die Veresterungsreaktion katalysierenden Enzyms in Abwesenheit eines Lösungsmittels bei einer Reaktionstemperatur von 10 bis 150 °C.

Die erfindungsgemäß herstellbaren Ester können in Kontaktlinsen oder als Vernetzer oder Haftverbesserer für Dispersionen, die vorzugsweise als Klebstoffe, Anstrichmittel oder Textil-, Leder- oder Papierhilfsmittel und in härtbaren Beschichtungen eingesetzt werden, verwendet werden.

Das erfindungsgemäße Verfahren wird im Folgenden detailliert beschrieben:
Durch das erfindungsgemäße Verfahren können Ester der (Meth)acrylsäure oder einem Ester davon mit Glycerincarbonat hergestellt werden. Dem Fachmann ist die Gruppe chemischer Verbindungen der Ester bekannt. Dabei wird in einer organischen Carbonsäure das Proton der Carbonsäure-Funktion durch einen Kohlenstoff enthaltenden Rest ersetzt.

Das erfindungsgemäße Verfahren umfasst die Reaktion von (Meth)acrylsäure oder einem Ester davon mit Glycerincarbonat.

In dem erfindungsgemäßen Verfahren können als Edukte im Allgemeinen (Meth)acrylsäure und alle dem Fachmann bekannten Ester davon eingesetzt werden. Erfindungsgemäß wird der Begriff (Meth)acrylsäure als Sammelbegriff für Acrylsäure und Methacrylsäure verwandt, d. h. der Begriff beschreibt Acrylsäure, Methacrylsäure oder Mischungen davon.

Erfindungsgemäß bevorzugt als Edukte eingesetzte Ester der (Meth)acrylsäure sind die entsprechenden Ester von Acrylsäure, d. h. Acrylate, oder von Methacrylsäure, d h. Methacrylate. Werden in dem erfindungsgemäßen Verfahren Ester von (Meth)acrylsäure eingesetzt, so sind diese Ester in dem vom Alkohol abstammenden Teil des Moleküls bevorzugt gesättigt, d. h. es liegen keine ungesättigten C-C-Doppel- oder Dreifachbindungen vor.

In einer besonders bevorzugten Ausführungsform des erfindungsgemäßen Verfahrens entspricht die als Ausgangsverbindung eingesetzte (Meth)acrylsäure oder ein Ester davon der allgemeinen Formel (I) 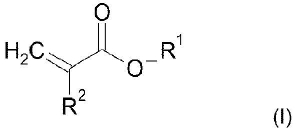 worin R¹ und R² unabhängig voneinander die folgenden Bedeutungen haben:
- R¹: Wasserstoff, Methyl, Ethyl, Propyl, beispielsweisen-Propyl,
- R²: Wasserstoff, aliphatischer, linearer oder verzweigter, gesättigter oder ungesättigter, gegebenenfalls Heteroatome ausgewählt aus N, O, P, S und/oder gegebenenfalls wenigstens eine funktionelle Gruppe enthaltender Kohlenwasserstoffrest mit insgesamt 1 bis 24 C-Atomen oder cyclischer, gesättigter oder ungesättigter, gegebenenfalls Heteroatome ausgewählt aus N, O, P, S und/oder gegebenenfalls wenigstens eine funktionelle Gruppe enthaltender Kohlenwasserstoffrest mit insgesamt 3 bis 24 C-Atomen oder substituierter oder unsubstituierter, gegebenenfalls Heteroatome ausgewählt aus N, O, P, S enthaltender aromatischer Kohlenwasserstoffrest mit insgesamt 5 bis 24 C-Atomen mit oder ohne funktionelle Gruppen, beispielsweise CN oder SO₃.

Gegebenenfalls an R² vorliegende Substituenten sind beispielsweise Alkylketten mit 1 bis 6 C-Atomen. Gegebenenfalls an R² als Substituenten vorliegende funktionelle Gruppen sind beispielsweise Hydroxy-, Amino-, Keto-, Carbonyl-, Halogenid-, Cyano-, Isocyano- und Sulfat-Gruppen. In einer bevorzugten Ausführungsform sind die als R² vorliegenden Reste nicht substituiert.

In der allgemeinen Formel (I) ist R¹ bevorzugt ausgewählt aus Wasserstoff oder Methyl.

In der allgemeinen Formel (I) ist R² bevorzugt ausgewählt aus Wasserstoff oder Methyl.

Die vorliegende Erfindung betrifft daher bevorzugt das erfindungsgemäße Verfahren, wobei als Derivat der (Meth)acrylsäure ein Ester, vorzugsweise ein C₁₋₁₂-Alkyl(meth)acrylat, besonders bevorzugt C₁₋₆-Alkyl(meth)acrylat, insbesondere ein Methyl-, Ethyl- oder Propylester, eingesetzt wird.

Erfindungsgemäß werden besonders bevorzugt Verbindungen der allgemeinen Formel eingesetzt, in denen R¹ und R² jeweils Wasserstoff (Acrylsäure), R¹ Wasserstoff und R² Methyl (Methacrylsäure), R¹ Methyl und R² Wasserstoff (Acrylsäuremethylester, Methylacrylat) oder R¹ und R² jeweils Methyl (Methacrylsäuremethylester, Methylmethacrylat) bedeuten.

Erfindungsgemäß können auch Mischungen eingesetzt werden, die zwei oder mehr Verbindungen der allgemeinen Formel (I) enthalten.

Verbindungen der allgemeinen Formel (I) bzw. deren Mischungen können nach den dem Fachmann bekannten Verfahren hergestellt werden oder sind kommerziellerhältlich.

In dem erfindungsgemäßen Verfahren wird neben (Meth)acrylsäure oder einem Ester davon auch Glycerincarbonat eingesetzt. Glycerincarbonat ist dem Fachmann an sich bekannt und im Folgenden als Verbindung (II) dargestellt:

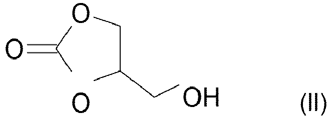

Glycerincarbonat ist kommerziell erhältlich und kann nach den dem Fachmann bekannten Verfahren, beispielsweise durch Umsetzung von Glycerin mit Harnstoff (EP955298), cyclischen Carbonaten wie Ethylencarbonat oder Propylencarbonat (EP0739888), linearen Carbonaten wie z. B. Diethylcarbonat oder Dimethylcarbonat (WO2010043581), erhalten werden. Möglich ist auch die Umsetzung von Glycerin mit Kohlenmonoxid (EP0582201), Phosgen oder Kohlendioxid (WO2011042288) sowie Glycidol mit Kohlendioxid (WO 2010106324).

In einer bevorzugten Ausführungsform des erfindungsgemäßen Verfahrens beträgt das molare Verhältnis von (Meth)acrylsäure oder einem Ester davon zu Glycerincarbonat 2:1, bis 30:1, besonders bevorzugt 4:1, bis 20:1 ganz besonders bevorzugt 5:1 bis 10:1. Das Verhältnis von (Meth)acrylsäure oder einem Ester davon zu Glycerincarbonat beträgt im Allgemeinen höchstens 100:1.

Durch das hohe Verhältnis von (Meth)acrylsäure oder einem Ester davon zu Glycerincarbonat kann auf ein Schleppmittel verzichtet werden, da die (Meth)acrylsäure oder das Derivat davon als Schleppmittel wirkt.

Das erfindungsgemäße Verfahren wird bei einer Reaktionstemperatur von 10 bis 100 °C durchgeführt. In einer bevorzugten Ausführungsform beträgt die Reaktionstemperatur 20 bis 90 °C, besonders bevorzugt 30 bis 70 °C, ganz besonders bevorzugt 40 bis 60 °C.

Das erfindungsgemäße Verfahren wird in Gegenwart wenigstens eines die Veresterungsreaktion katalysierenden Enzyms durchgeführt. Als Enzyme können erfindungsgemäß bevorzugt Hydrolasen [EC 3.x.x.x], insbesondere Esterasen [EC 3.1.x.x.] und Proteasen [EC 3.4.x.x], eingesetzt werden. Bevorzugt sind die Carboxylester-Hydrolasen [EC 3.1.1.x]. Besonders bevorzugt werden Lipasen als Hydrolasen eingesetzt. Insbesondere werden Lipasen aus Achromobacter sp., Aspergillus sp., Burholderia sp., Candida sp., Mucor sp., Penicillium sp., Pseudomonas sp., Rhizopus sp., Thermomyces sp. oder Schweinepankreas verwendet. Die Enzyme und ihre Funktionen werden beispielsweise in Römpp Online, 2002, "Hydrolasen", "Lipasen" und "Proteasen" beschrieben.

Besonders bevorzugt wird erfindungsgemäß eine Lipase aus Candida Antarctica (Candida Antarctica Lipase B) als Enzym eingesetzt.

Das wenigstens eine Enzym wird erfindungsgemäß in einer Menge von 0,1 bis 20 Gew.-%, bevorzugt 0,5 bis 10 Gew.-%, besonders bevorzugt 0,7 bis 5 Gew.-%, jeweils bezogen auf das in der Reaktionsmischung vorliegende Glycerincarbonat, eingesetzt.

Das wenigstens eine Enzym kann erfindungsgemäß mobilisiert oder immobilisiert eingesetzt werden. Erfindungsgemäß bevorzugt werden immobilisierte, d. h. auf einem Träger vorliegende Enzyme eingesetzt. Geeignete Trägermaterialien sind dem Fachmann an sich bekannt, insbesondere werden organische, polymere Trägermaterialien eingesetzt, um das in dem erfindungsgemäßen Verfahren eingesetzte wenigstens eine Enzym zu immobilisieren.

Die vorliegende Erfindung betrifft daher bevorzugt das erfindungsgemäße Verfahren, wobei das wenigstens eine Enzym auf einem Trägermaterial vorliegt.

Das Enzym ist in dem erfindungsgemäßen Verfahren bevorzugt auf einem geeigneten Träger immobilisiert. Dabei existieren im Allgemeinen fünf klassische Methoden der Immobilisierung von Enzymen, nämlich die Adsorption, die kovalente Bindung, der Membraneinschluss, der Geleinschluss und die Quervernetzung. Dabei können unterschiedliche Trägermaterialien eingesetzt werden, wobei die chemischen Wechselwirkungen der Trägeroberfläche mit dem Enzym so angepasst sein müssen, dass keine unerwünschten Nebenwirkungen, wie z. B. Inaktivierung, entstehen. Als feste Träger eignen sich prinzipiell verschiedene anorganische und organische Materialien, letztere können natürlichen oder synthetischen Ursprungs sein. Anorganische Träger weisen meistens eine hohe Druckstabilität auf, während organische Träger eine gute chemische Stabilität zeigen. Als anorganische Träger werden überwiegend poröse Materialien auf der Basis von Silicium- oder Aluminiumoxiden bzw. Gemischen daraus eingesetzt. Natürliche organische Träger sind beispielsweise Polysaccharide, wie z. B. Cellulose, Stärke, Dextran, Agarose und Chitin. Aber auch Proteine, wie Kollagen, Gelatine und Albumin, kommen zur Anwendung. Als synthetische organische Polymere dienen Poly(meth)acrylate, Polyacrylamide, Vinyl- und Allylpolymere, Polyester oder Polyamide.

Ein Beispiel für ein besonders bevorzugt eingesetztes Trägermaterial ist ein makroporöses, Divinylbenzol-vernetztes Polymer in sphärischer Perlenform auf Basis von Methacrylat. Dieses bevorzugt eingesetzte Trägermaterial weist eine Korngröße (D₈₀) von 0,3 bis 1,5 mm, bevorzugt 0,31 bis 1,2 mm, und eine effektive Größe von 0,3 bis 0,6 mm, bevorzugt 0,3 bis 0,5 mm, auf. Die Dichte des bevorzugt eingesetzten Trägermaterials beträgt beispielsweise 1,0 bis 1,5 g/ml, bevorzugt 1,02 bis 1,1 g/ml. Der Wassergehalt des bevorzugt eingesetzten Trägermaterials beträgt beispielsweise 40 bis 80 Gew.-%, bevorzugt 50 bis 70 Gew.-%.

Die BET-Oberfläche des erfindungsgemäß bevorzugt eingesetzten organischen Trägermaterials beträgt beispielsweise 100 bis 200 m²/g, bevorzugt 110 bis 150 m²/g.

Das Porenvolumen des erfindungsgemäß bevorzugt eingesetzten Trägermaterials beträgt beispielsweise 0,2 bis 1,0 cm³/g, bevorzugt 0,4 bis 0,8 cm³/g.

Der Porendurchmesser der in dem erfindungsgemäß bevorzugt eingesetzten Trägermaterial vorliegenden Poren beträgt beispielsweise 5 bis 50 nm, bevorzugt 10 bis 30 nm.

Besonders bevorzugt werden in dem erfindungsgemäßen Verfahren Enzyme eingesetzt, die bereits auf einem geeigneten Träger immobilisiert sind. Derartige immobilisierte Enzyme, bevorzugt Lipasen, sind unter dem Handelsnamen Novozym® 435 (Lipase aus Candida antarctica B) von der Firma Novozymes erhältlich.

Gemäß der bevorzugten Ausführungsform, bei der das wenigstens eine Enzym auf einem Trägermaterial vorliegt, liegt es im Allgemeinen in einer Menge von 1 bis 20 Gew.-%, bevorzugt 2 bis 15 Gew.-%, besonders bevorzugt 5 bis 12 Gew.-%, jeweils bezogen auf die Summe aus Enzym und Trägermaterial, vor.

Die im erfindungsgemäßen Verfahren eingesetzten Enzyme, die die Veresterungsreaktion katalysieren, können auch in situ im Verfahren aus entsprechenden Organismen erhalten werden. Als Organismen kommen dazu alle natürlich vorkommenden oder gentechnisch veränderten Mikroorganismen, einzelligen Lebewesen oder Zellen in Betracht, die die Umesterung oder Veresterung mittels einer Hydrolase [EC 3.x.x.x], bevorzugt einer Esterase [EC 3.1.x.x.] oder Protease [EC 3.4.x.x], bevorzugt einer Carboxylesterhydrolase [EC 3.1.1.x] und insbesondere einer Lipase, katalysieren. Es sind alle dem Fachmann bekannten Organismen einsetzbar, die Hydrolasen beinhalten. Bevorzugt werden Organismen eingesetzt, die als Hydrolasen Lipasen umfassen. Insbesondere finden Achromobacter sp., Aspergillus sp., Burholderia sp., Candida sp., Mucor sp., Penicillium sp., Pseudomonas sp., Rhizopus sp., Thermomyces sp. und Zellen aus Schweinepankreas Verwendung. Dabei kann es sich um die unveränderten Organismen selbst oder um gentechnisch veränderte Organismen handeln, die die Enzyme ursprünglich nicht oder nur ungenügend stark exprimieren und erst nach Veränderung eine genügend hohe Enzym-Aktivität und Produktivität aufweisen. Ferner können die Organismen durch die gentechnische Veränderung an die Reaktionsbedingungen und/oder Kultivierungsbedingungen angepasst werden.

Das erfindungsgemäße Verfahren wird in Abwesenheit eines Lösungsmittels durchgeführt. Im Rahmen der vorliegenden Erfindung ist unter "in Abwesenheit eines Lösungsmittels" zu verstehen, dass neben den vorhandenen Substraten, d. h. (Meth)acrylsäure oder ein Derivat davon und Glycerincarbonat, dem wenigstens einen Enzym, gegebenenfalls wenigstens einem Stabilisator und gegebenenfalls wenigstens einem Trockenmittel, kein weiteres organisches oder wässriges Lösungsmittel in der Reaktionsmischung vorhanden ist.

Im Laufe der erfindungsgemäßen Veresterungs- bzw. Umesterungsreaktion werden als Reaktionsprodukte beispielsweise Wasser bei Einsatz entsprechender (Meth)acrylsäuren oder Alkohole, beispielsweise Methanol, Ethanol, Propanol, bei Einsatz der entsprechenden Ester, insbesondere (Meth)acrylsäureester, gebildet. Um die Reaktionsgeschwindigkeit durch Verlagerung des chemischen Gleichgewichts weiter zu erhöhen, ist es erfindungsgemäß bevorzugt, dass das während der Reaktion gebildete Wasser oder die entsprechenden Alkohole kontinuierlich entfernt werden.

Das kontinuierliche Entfernen des während der Reaktion gebildeten Wassers oder der entsprechenden Alkohole kann im Allgemeinen nach allen dem Fachmann bekannten Methoden erfolgen, beispielsweise Einsatz eines Trockenmittels, einer hydrophoben Membran, destillative Abtrennung und Kombinationen davon.

Erfindungsgemäß bevorzugt betrifft die vorliegende Erfindung daher das erfindungsgemäße Verfahren, wobei es in Gegenwart eines Trockenmittels durchgeführt wird. Erfindungsgemäß einzusetzende Trockenmittel sind dem Fachmann an sich bekannt und beispielsweise ausgewählt aus der Gruppe bestehend aus Molsieben, Calciumchlorid, Blaugel, Magnesiumsulfat und Mischungen davon.

In einer bevorzugten Ausführungsform des erfindungsgemäßen Verfahrens, insbesondere wenn dieses in einem größeren Maßstab, beispielsweise Pilot Plant oder industrieller Maßstab, durchgeführt wird, werden während der Reaktion Wasser, Alkohole, beispielsweise Methanol, Ethanol und/oder Propanol, oder Mischungen davon destillativ entfernt. Entsprechende Vorrichtungen sind dem Fachmann an sich bekannt, beispielsweise Destillationskolonnen.

In einer bevorzugten Ausführungsform des erfindungsgemäßen Verfahrens wird es in Gegenwart wenigstens eines Stabilisators durchgeführt. Weiter bevorzugt wird ein Stabilisator eingesetzt, um die Polymerisationsneigung der eingesetzten Edukte, d. h. der (Meth)acrylsäure oder einem Derivat davon, bzw. der während der Reaktion entstehenden Produkte, d. h. der Ester von Glycerincarbonat mit (Meth)acrylsäure oder einem Derivat davon, zu unterbinden bzw. zu unterdrücken.

Geeignete Stabilisatoren bzw. Polymerisationsinhibitoren können beispielsweise sein N-Oxide (Nitroxyl- oder N-Oxyl-Radikale, also Verbindungen, die wenigstens eine >N-O-Gruppe aufweisen), wie z. B. 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethyl-piperidin-N-oxyl, 2,2,6,6-Tetramethylpiperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl; ein- oder mehrwertige Phenole, die ggf. eine oder mehrere Alkylgruppen aufweisen, wie z. B. Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie z. B. Hydrochinon, Hydrochinonmonomethylether, 2-Methylhydrochinon oder2,5-Di-tert.-Butylhydrochinon; Hydroxyphenole, wie beispielsweise Brenzcatechin (1,2-Dihydroxybenzol) oder Benzochinon; Aminophenole, wie z. B. p-Aminophenol; Nitrosophenole, wie z. B. p-Nitrosophenol; Alkoxyphenole, wie beispielsweise 2-Methoxyphenol (Guajacol,Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol; Tocipherole, wie z. B. α-Tocopherol sowie 2,3-Dihydro-2,2-dimethyl-7-hydroxybenzofuran (2,2-Dimethyl-7-hydroxycumaran), aromatische Amine, wie z. B. N,N-Diphenylamin oder N-Nitroso-diphenylamin; Phenylendiamine, wie z. B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z. B. N,N'-Dimethyl-p-phenylendiamin oder N,N'-Diethyl-p-phenylendiamin, Hydro-xylamine, wie z.B. N,N-Diethylhydroxylamin, Imine, wie z. B. Methylethylimin oder Methylen violett, Sulfonamide, wie z. B. N-Methyl-4-toluolsulfonamid oder N-tert.-Butyl-4-toluolsulfonamid, Oxime, wie Aldoxime, Ketoxime oder Amidoxime, wie z. B. Diethylketoxim, Methylethylketoxim oder Salicyladoxim, phosphorhaltige Verbindungen, wiez. B. Triphenylphosphin, Triphenylphosphit, Triethylphosphit, hypophsophorige Säure oder Alkylester der phosphorigen Säuren; schwefelhaltige Verbindungen wie z. B. Diphenylsulfid oder Phenothiazin; Metallsalze, wie Kupfer- oder Mangan-,Cer-, Nickel-, Chromsalze, beispielsweise Metallchloride, -sulfate, -salicylate, -tosylate, -acrylate oder -acetate, wie z. B. Kupferacetat, Kupfer(II)chlorid, Kupfersalicylat, Cer(III)acetat oder Cer(III)ethylhexanoat, oder Gemische davon.

Besonders geeignete Stabilisatoren für das erfindungsgemäße Verfahren sind Hydrochinon, Hydrochinonmonomethylether (MEHQ), Phenothiazin, 4-Hydroxy-2,2,6,6-tetra-methylpiperidin-N-oxyl, 4-Oxo-2,2,6,6-tetramethylpiperidin-N-oxyl, 2-tert.-Butylphenol, 4-tert.-Butylphenol, 2,4-Di-tert.-butylphenol, 2-tert.-Butyl-4-methylphenol, 6-tert.-Butyl-2,4-dimethyl-phenol, 2,6-Di-tert.-butyl-4-methylphenol, 2-Methyl-4-tert.-butylphenol, hypophosphorige Säure, Kupferacetat, Kupfer(II)chlorid, Kupfersalicylat und Cer(III)acetat. Insbesondere bevorzugte Stabilisatoren sind Hydrochinonmonomethylether (MEHQ) und/oder Phenothiazin (PTZ).

Im Allgemeinen werden, jeweils bezogen auf die ungesättigten Monomere, je Einzelsubstanz 1 bis 10 000 ppm, bevorzugt 10 bis 5 000 ppm, besonders bevorzugt 30 bis 2 500 ppm und insbesondere 50 bis 1 500 ppm, eines geeigneten Polymerisationsinhibitors eingesetzt.

Das erfindungsgemäße Verfahren kann im Allgemeinen bei einem Druck von 0,01 bis 1,5 bar (a) durchgeführt werden. Das erfindungsgemäße Verfahren wird in einer bevorzugten Ausführungsform bei Atmosphärendruck durchgeführt.

Weiter bevorzugt wird das Verfahren bei einem Druck durchgeführt, der unterhalb Atmosphärendruck liegt, d. h. bei einem Druck von 0,01 bis ca. 0,9 bar (a). In der bevorzugten Ausführungsform, dass während des erfindungsgemäßen Verfahrens das entstehende Wasser bzw. niedermolekulare Alkohole kontinuierlich destillativ entfernt werden, wird das Verfahren insbesondere bei einem Druck von 0,01 bis 0,5 bar (a) durchgeführt, um den Siedepunkt der entstehenden niedermolekularen Verbindungen weiter herabzusenken.

Nach erfolgter Reaktion kann die erhaltene Reaktionsmischung im Allgemeinen nach allen dem Fachmann bekannten Methoden aufgearbeitet werden, beispielsweise Filtration, destillatives Entfernen von im Überschuss vorhandener Substrate etc.

Die erfindungsgemäß erhaltenen Produkte entsprechen der allgemeinen Formel (III) 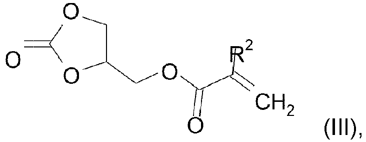 worin R² die gleichen Bedeutungen aufweist wie bezüglich der Verbindung der allgemeinen Formel (I) ausgeführt.

Bevorzugt ist in der Verbindung der allgemeinen Formel (III) R² Wasserstoff oder Methyl.

Die erfindungsgemäß hergestellten Ester, insbesondere die Verbindungen der allgemeinen Formel (III), zeichnen sich durch eine besonders hohe Reinheit aus. Diese hohe Reinheit macht es möglich, dass die erfindungsgemäß hergestellten Verbindungen ohne weitere Aufreinigung eingesetzt werden können

In einer weiteren Ausführungsform des erfindungsgemäßen Verfahrens wird nach der Reaktion der erhaltene Glycerincarbonat-(Meth)acrylsäure-Ester oder ein Derivat davon in Glycerinmono-(Meth)acrylsäure-Ester oder ein Derivat davon überführt.

Dieser optionale Schritt des erfindungsgemäßen Verfahrens umfasst dass Abspalten der Carbonat-Schutzgruppe um so die beiden Hydroxygruppen in freier Form zu erhalten. Das Produkt dieses optionalen Spaltungsschrittes ist ein Glycerin-mono-(Meth)acrylsäure-Ester oder ein Derivat davon, im Folgenden als allgemeine Formel (IV) dargestellt: 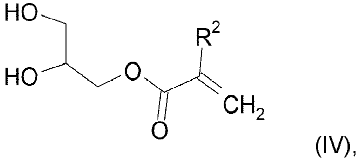 worin R² die gleichen Bedeutungen aufweist wie bezüglich der Verbindung der allgemeinen Formel (I) ausgeführt.

Bevorzugt ist in der Verbindung der allgemeinen Formel (IV) R² Wasserstoff oder Methyl.

Der optionale Spaltungsschritt in dem erfindungsgemäßen Verfahren kann im Allgemeinen nach allen dem Fachmann bekannten Methoden erfolgen, beispielsweise durch milde alkalische Hydrolyse. Geeignete Methoden finden sich in T. W. Greene, Protective Groups in Organic Synthesis, Second Edition 1991, John Wiley & Sons Inc., S. 108, 109, 140, 141.

Die erfindungsgemäß durch die Abspaltung der Carbonat-Schutzgruppe hergestellten Ester zeichnen sich gegenüber entsprechenden Verbindungen, hergestellt durch Verfahren des Standes der Technik, durch explizite Monofunktionalisierung, also die Abwesenheit von Glycerindi(meth)acrylaten, aus.

Insbesondere beschreibt die vorliegende Erfindung die folgenden Verbindungen:

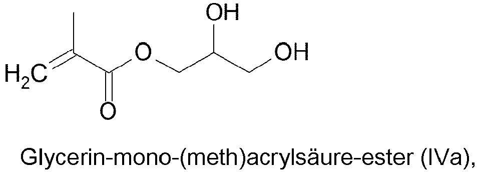

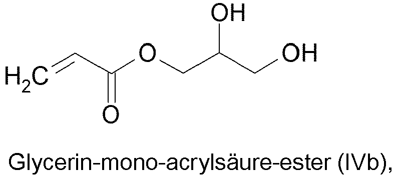

Besonders bevorzugt beschreibt die vorliegende Erfindung Glycerin-mono-(meth)acrylsäure-ester. Durch das erfindungsgemäße Verfahren ist es möglich, Glycerin-mono-(meth)acrylsäure-ester in einer ganz besonders hohen Reinheit zu erhalten, d. h. im Wesentlichen frei von Glycerin-di-(metha)crylsäure-ester. In einer bevorzugten Ausführungsform ist es erfindungsgemäß möglich, Glycerin-mono-(meth)acrylsäure-ester in einer hohen Reinheit zu erhalten.

Die vorliegende Erfindung beschreibt daher auch die Verwendung eines erfindungsgemäß hergestellten Esters, insbesondere von Glycerin-mono-(meth)acrylsäure-ester, in Kontaktlinsen oder als Vernetzer oder Haftverbesserer für Dispersionen, die vorzugsweise als Klebstoffe, Anstrichmittel oder Textil-, Leder- oder Papierhilfsmittel und in härtbaren Beschichtungen eingesetzt werden.

### Beispiele

In allen Versuchen wird als Enzym Novozym® 435 eingesetzt. Dies ist eine Lipase aus Candida Antarctica auf einem makroporösen, Divinylbenzol-vernetzten Polymer in sphärischer Perlenform auf Basis von Methacrylat. Das Trägermaterial weist eine Korngröße (D₈₀) von 0,315 bis 1,0 mm, und eine effektive Größe von 0,32 bis 0,45 mm auf. Die Dichte beträgt 1,06 g/ml, der Wassergehalt beträgt 55 bis 65 Gew.-%. Die BET-Oberfläche des Trägermaterials beträgt 130 m²/g. Das Porenvolumen des Trägermaterials beträgt 0,5 cm³/g. Der Porendurchmesser der in dem Trägermaterial vorliegenden Poren beträgt 15 nm.

### Beispiel 1 (ohne Molekularsieb):

In einer 50 ml Schottflasche wurden Glycerincarbonat (4,0 g, 0,034 mol), Methylacrylat (15,0 g, 0,174 mol), MeHQ (ca. 400 ppm), Novozym® 435 (0,4 g, 10 Gew.-%) eingewogen. Der Ansatz wurde bei 40 °C im Wasserbadschüttler geschüttelt. Der Umsatz wurde per GC kontrolliert. Die Ergebnisse nach 2 h, 4 h und 8 h sind in Tabelle 1 gezeigt:

**Tabelle 1:**

| | 2 h [Flächen-%] | 4 h [Flächen-%] | 8 h [Flächen-%] |
|---|---|---|---|
| Glycerincarbonat | 50,50 | 45,50 | 44,50 |
| Glycerincarbonat-acrylsäure-ester | 49,50 | 54,50 | 55,50 |

### Beispiel 2 (mit Molekularsieb):

In einer 50 ml Schottflasche wurden Glycerincarbonat (4,0 g, 0,034 mol), Methylacrylat 10 (15,0 g, 0,174 mol), MeHQ (ca. 400 ppm), Novozym® 435 (0,4 g, 10 Gew.-%) und 5 A Molekularsieb (9 g) eingewogen. Der Ansatz wurde bei 40 °C im Wasserbadschüttler geschüttelt. Der Umsatz wurde per GC kontrolliert. Die Ergebnisse nach 2 h, 4 h und 8 h sind in Tabelle 2 gezeigt:

**Tabelle 2:**

| | 2 h [Flächen-%] | 4 h [Flächen-%] | 8 h [Flächen-%] |
|---|---|---|---|
| Glycerincarbonat | 58,50 | 17,10 | <1 |
| Glycerincarbonat acrylsäure-ester | 41,50 | 82,90 | >99 |

### Beispiel 3 (Up-scale-Experiment):

In einen 10 L HWS Miniplantreaktor wurden Glycerincarbonat (1000 g, 8,46 mol), Methylacrylat (3645 g, 42,34 mol), MeHQ (400 mg) und Novozym® 435 (75 g, 7,5 Gew.-%) eingewogen. Es wurde bei 40 °C ,195 mbar und 140 Upm gerührt. Methanol wurde durch Destillation von dem Azeotrop Methanol/Methylacrylat kontinuierlich entfernt. Der Umsatz wurde per GC kontrolliert.

Die Ergebnisse nach bestimmten Zeiten sind in Tabelle 3 gezeigt:

**Tabelle 3:**

| Zeit [h] | Zeit [min.] | Edukt [Flächen-%, RT-Zeit: 6,048'] | Produkt [Flächen-%,RT-Zeit: 6,867'] |
|---|---|---|---|
| 0 | 0 | 94,1 | 0 |
| 2,00 | 120 | 40,99 | 59 |
| 4,00 | 240 | 27,87 | 72,12 |
| 6,00 | 360 | 18,97 | 81,02 |
| 9,00 | 540 | 13,73 | 86,26 |
| 11,00 | 660 | 11,54 | 88,45 |
| 14,00 | 840 | 9,18 | 90,81 |
| 16,00 | 960 | 8,21 | 91,78 |
| 18,00 | 1080 | 6,41 | 93,58 |
| 20,00 | 1200 | 6,05 | 93,94 |
| 23,00 | 1380 | 4,27 | 95,72 |
| 25,00 | 1500 | 3,63 | 96,36 |

### Beispiel 4 (Up-scale-Experiment):

In einen 10 L HWS Miniplantreaktor wurden Glycerincarbonat (600 g, 5,08 mol), Methylacrylat (4374 g, 50,81 mol), MeHQ (250 mg) und Novozym® 435 (45 g, 7,6 Gew.-%) als Festbett eingewogen. Die Reaktionsmischung wurde kontinuierlich durch dieses Festbett gepumpt bis die Reaktion beendet war. Die Bedingungen waren 50 °C ,195 mbar und 140 Upm. Methanol wurde durch Destillation von dem Azeotrop Methanol/Methylacrylat kontinuierlich entfernt. Der Umsatz wurde per GC kontrolliert. Die Ergebnisse nach bestimmten Zeiten sind in Tabelle 4 gezeigt:

**Tabelle 4:**

| Zeit [h] | Zeit [min] | Edukt [Flächen-%, RT-Zeit: 6,048'] | Produkt [Flächen-%, RT-Zeit: 6,867'] |
|---|---|---|---|
| 0,00 | 0 | 96,34 | 0 |
| 1,00 | 60 | 32,91 | 67,08 |
| 2,00 | 120 | 24,2 | 75,79 |
| 4,00 | 240 | 13,61 | 86,38 |
| 5,00 | 300 | 13,69 | 86,3 |
| 6,00 | 360 | 8,85 | 91,14 |
| 8,00 | 480 | 6,01 | 93,98 |
| 10,00 | 600 | 3,67 | 96,32 |
| 12,00 | 720 | 3,30 | 96,69 |
| 14,00 | 840 | 2,81 | 97,48 |
| 15,00 | 900 | 1,42 | 98,57 |

## Patentansprüche

1. Verfahren zur Herstellung von Estern der (Meth)acrylsäure
durch Umsetzung von (Meth)acrylsäure oder einem Ester davon mit Glycerincarbonat in Gegenwart wenigstens eines die Veresterungsreaktion katalysierenden Enzyms in Abwesenheit eines Lösungsmittels bei einer Reaktionstemperatur von 10 bis 150 °C.

2. Verfahren nach Anspruch 1, **dadurch gekennzeichnet, dass** die Reaktionstemperatur 10 bis 100 °C, bevorzugt 20 bis 90 °C, besonders bevorzugt 40 bis 60 °C, beträgt.

3. Verfahren nach Anspruch 1 oder 2, **dadurch gekennzeichnet, dass** eine Lipase aus Candida antarctica als Enzym eingesetzt wird.

4. Verfahren nach einem der Ansprüche 1 bis 3, **dadurch gekennzeichnet, dass** das wenigstens eine Enzym auf einem Trägermaterial vorliegt.

5. Verfahren nach einem der Ansprüche 1 bis 4, **dadurch gekennzeichnet, dass** als Ester der (Meth)acrylsäure ein C₁₋₁₂-Alkyl-Ester, insbesondere ein Methyl-, Ethyl- oder Propylester, eingesetzt wird.

6. Verfahren nach einem der Ansprüche 1 bis 5, **dadurch gekennzeichnet, dass** das Verfahren in Gegenwart eines Trockenmittels durchgeführt wird.

7. Verfahren nach einem der Ansprüche 1 bis 6, **dadurch gekennzeichnet, dass** es in Gegenwart wenigstens eines Stabilisators durchgeführt wird.

8. Verfahren nach einem der Ansprüche 1 bis 7, **dadurch gekennzeichnet, dass** während der Reaktion Wasser, Alkohole oder Mischungen davon destillativ entfernt werden.

9. Verfahren nach einem der Ansprüche 1 bis 8, **dadurch gekennzeichnet, dass** das molare Verhältnis von (Meth)acrylsäure oder einem Ester davon zu Glycerincarbonat 2:1 bis 30:1 beträgt.

10. Verfahren nach einem der Ansprüche 1 bis 9, **dadurch gekennzeichnet, dass** nach der Reaktion der erhaltene Glycerincarbonat-(Meth)acrylsäure-Ester in Glycerin-mono-(Meth)acrylsäure-Ester überführt wird.

## Claims

1. A process for preparing an ester of (meth)acrylic acid by reaction of (meth)acrylic acid or an ester thereof with glycerol carbonate at a reaction temperature of 10 to 150°C without a solvent in the presence of at least one enzyme catalyzing the esterification reaction.

2. The process according to claim 1 wherein the reaction temperature is in the range from 10 to 100°C, preferably in the range from 20 to 90°C and more preferably in the range from 40 to 60°C.

3. The process according to claim 1 or 2 wherein the enzyme used is a lipase from Candida antarctica.

4. The process according to any one of claims 1 to 3 wherein the at least one enzyme is present on a carrier material.

5. The process according to any one of claims 1 to 4 wherein the (meth)acrylic acid ester used is a C₁₋₁₂-alkyl ester, especially a methyl, ethyl or propyl ester.

6. The process according to any one of claims 1 to 5 in the presence of a drier.

7. The process according to any one of claims 1 to 6 in the presence of at least one stabilizer.

8. The process according to any one of claims 1 to 7 wherein water, alcohols or mixtures thereof are removed during the reaction by distillation.

9. The process according to any one of claims 1 to 8 wherein the molar ratio of (meth)acrylic acid or an ester thereof to glycerol carbonate is in the range from 2:1 to 30:1.

10. The process according to any one of claims 1 to 9 wherein the resulting glycerol carbonate (meth)acrylate is converted into glycerol mono(meth)acrylate after the reaction.

## Revendications

1. Procédé pour la préparation d'esters de l'acide (méth)acrylique par transformation d'acide (méth)acrylique ou d'un ester de celui-ci avec un carbonate de glycérine en présence d'au moins une enzyme catalysant la réaction d'estérification en absence d'un solvant à une température de réaction de 10 à 150 °C.

2. Procédé selon la revendication 1, **caractérisé en ce que** la température de réaction est de 10 à 100 °C, préférablement 20 à 90 °C, particulièrement préférablement 40 à 60 °C.

3. Procédé selon la revendication 1 ou 2, **caractérisé en ce qu'**une lipase de Candida Antartica est utilisée comme enzyme.

4. Procédé selon l'une quelconque des revendications 1 à 3, **caractérisé en ce que** l'au moins une enzyme est présente sur un matériau de support.

5. Procédé selon l'une quelconque des revendications 1 à 4, **caractérisé en ce qu'**un ester de C₁₋₁₂-alkyle, en particulier un ester de méthyle, d'éthyle ou de propyle, est utilisé en tant qu'ester de l'acide (méth)acrylique.

6. Procédé selon l'une quelconque des revendications 1 à 5, **caractérisé en ce que** le procédé est mis en œuvre en présence d'un dessicatif.

7. Procédé selon l'une quelconque des revendications 1 à 6, **caractérisé en ce qu'**il est mis en œuvre en présence d'un stabilisant.

8. Procédé selon l'une quelconque des revendications 1 à 7, **caractérisé en ce que** de l'eau, des alcools ou des mélanges de ceux-ci sont éliminés par distillation pendant la réaction.

9. Procédé selon l'une quelconque des revendications 1 à 8, **caractérisé en ce que** le rapport molaire d'acide (méth)acrylique ou d'un ester de celui-ci sur le carbonate de glycérine est de 2 : 1 à 30 : 1.

10. Procédé selon l'une quelconque des revendications 1 à 9, **caractérisé en ce qu'**après la réaction, l'ester d'acide (méth)acrylique de carbonate de glycérine obtenu est converti en monoester d'acide (méth)acrylique de glycérine.
